# EUROPEAN PATENT APPLICATION

(11) **EP 2 073 137 A2**
(43) Date of publication of application: **24.06.2009**
(21) Application number: 08251719.4
(22) Date of filing: 15.05.2008
(51) Int. Cl.: G06F 19/00

(54) **System and method of obtaining informed consent**

(30) Priority: 19.12.2007 US 960352
(71) Applicant: NCR Corporation, Dayton, Ohio 45479 (US)
(72) Inventor: Toleti, Chakravarthy S.K., Windermere FL 34786 (US); Toleti, Rajesh S.K., Windermere FL 34786 (US); Vempaty, Nageshwara R., Saratoga CA 95070 (US)
(74) Representative: MacLeod, Roderick William

(57) **Abstract**

A system and method of obtaining informed consent which reduces paperwork. The method includes obtaining electronic informed consent information associated with a medical procedure by a computer, presenting the informed consent information to a prospective participant of the medical procedure by the computer, and electronically storing an informed consent from the prospective participant by the computer. The system may include, for example, one or a plurality of a tablet computer, a desktop computer, or a kiosk.

## Description

In the US and other countries, experimental medical procedures affecting people must be conducted in accordance with established regulations. Experimental procedures may include drug protocols, experimental treatments, and other advances in medicine.

When a patient is considering enrolling in an experimental procedure, the patient typically completes an informed consent process. This process is important to anyone involved, including testing facilities, sponsors, manufacturers, healthcare institutions, and healthcare providers for regulatory and legal reasons.

The current process of obtaining an informed consent is paper-based. It involves presenting a prospective participant with a paper informed consent document, educating the prospective participant about the details in the document, and then asking the prospective participant to consent or not to consent to the procedure by signing the paper informed consent document.

A presenter of the paper informed consent document typically acts as a witness and also signs the document. The witness signs regardless of whether the prospective participant decides to participate or not to participate.

A system and method of obtaining informed consent is provided.

The method includes obtaining electronic informed consent information associated with a medical procedure by a computer, presenting the informed consent information to a prospective participant of the medical procedure by the computer, and electronically storing an informed consent from the prospective participant by the computer.

The system may include one or a plurality of computers, each computer may be, for example, a tablet computer, a desktop computer, or a kiosk.

According to a first aspect of the present invention there is provided a method of obtaining informed consent comprising:(a) obtaining electronic informed consent information associated with a medical procedure by a computer;(b) presenting the informed consent information to a prospective participant of the medical procedure by the computer; and(c) electronically storing an informed consent from the prospective participant by the computer.

According to a second aspect of the present invention there is provided a system for obtaining informed consents comprising: a computer for obtaining electronic informed consent information associated with a medical procedure, for presenting the informed consent information to a prospective participant of the medical procedure, and for electronically storing an informed consent from the prospective participant by the computer.
Preferably, step (c-1) comprises: (c-1-a) capturing the first and second signatures by a signature capture device coupled to the computer.

Preferably, the computer comprises a tablet computer with a touch screen and step (c-1) comprises: (c-1-a) capturing the first and second signatures by the touch screen of the tablet computer.
Preferably, step (b) comprises :(b-1) printing an electronic informed consent form by a printer coupled to a computer. Preferably, the method further comprises: (d) presenting additional information about the medical procedure to the prospective participant.
Preferably, the computer captures a first signature of the prospective participant and a second signature of a witness. Preferably, the computer comprises a tablet computer, a desktop computer or a kiosk.

Embodiments of the present invention will now be described, by way of example, with reference to the accompanying drawings, in which:
Fig. 1 is a block diagram of a healthcare provider system for obtaining informed consent from a patient.
Fig. 2 is a flowchart illustrating an example method of obtaining informed consent from the patient.

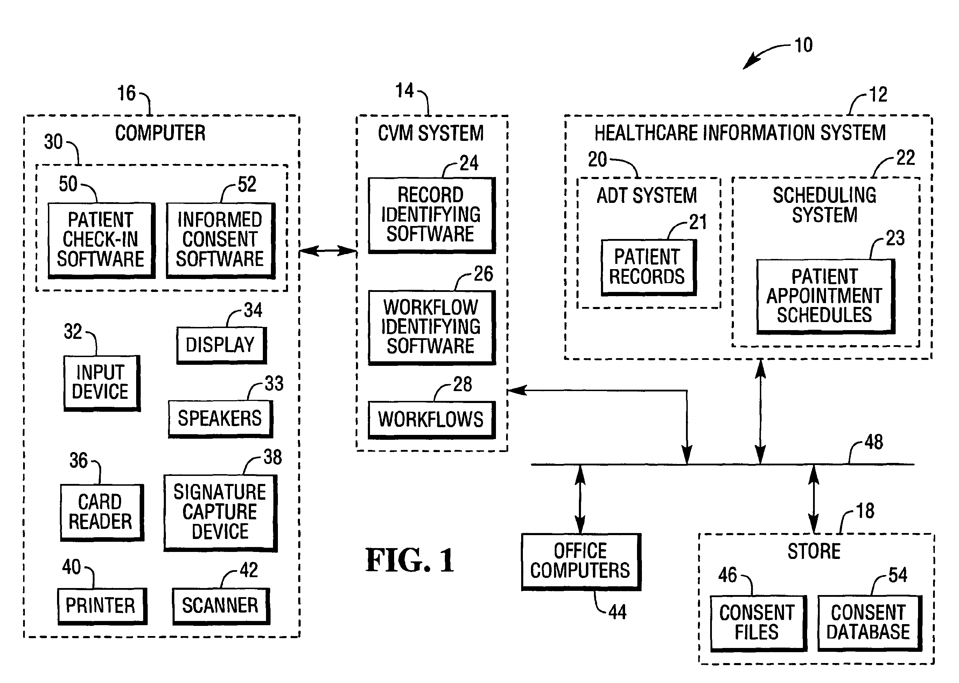

With reference to Fig. 1, healthcare system 10 includes healthcare information system 12, customer value management (CVM) system 14, computer 16, and data store 18. Healthcare information system 12, CVM system 14, and computer 16 include processors and memory for executing programs and storing data. Healthcare information system 12, CVM system 14, and store 18 may be combined into one or more computers.

Healthcare system 10 may be located at a healthcare provider office. The healthcare provider office may include a front desk, a lounge, and examination rooms. Healthcare information system 12 may alternatively be located external to the healthcare provider office.

Healthcare information system 12 stores patient information. Healthcare information system 12 may include admission, discharge, and transfer (ADT) system 20 and scheduling system 22.

ADT system 20 includes patient records 21. Patient records may include completed informed consent data.

Scheduling system 22 includes patient appointment schedules 23.

CVM system 14 executes record identifying software 24 which attempts to match patient records 21 to the patient identifying information presented by patients during check-in. Record identifying software 24 performs the matching process in a way that satisfies a local security policy. CVM system 14 may be located at the healthcare provider office or external to the healthcare provider office.

CVM system 14 additionally executes workflow identifying software 26. Workflow 28 may present a patient with requests for information and present questions or guidance based upon the answers.

An example workflow 28 includes obtaining a prospective patient's informed consent to participate in an experimental procedure.

The informed consent workflow obtains consent files 46 from store 18, including determining whether an informed consent for a medical procedure exists and returning the informed consent or an error to informed consent software 52.

Computer 16 executes 30 software applications to help automate processing tasks. Check-in software 50 displays prompts instructing patients to enter their identifying information, in accordance with the local security policy, and passes the identifying information to record identifying software 24 for analysis. If a patient is not already on file, then check-in software 50 displays prompts and records information about the patient.

Patients may update their information, i.e., read and write information, securely through patient check-in software 50. Patient information may be encrypted to make it secure for sending over network 48.

Informed consent software 52 automates an informed consent process. Informed consent software 52 interacts with CVM system 14 to obtain information from and store information in store 18.

Informed consent software 52 may complete an informed consent process over one session or over more sessions where the informed consent is presented over multiple sessions. Informed consent database 54 keeps track of portions completed and resumes subsequent sessions at the next logical point, eventually leading to the completion of the informed consent process.

Patient check-in software 50 and informed consent software 52 may be web applications. The patient accesses and enters information through a web browser executed by computer 16. Patient check-in software 50 and informed consent software 52 may reside in a server.

Computer 16 may be a tablet computer, desktop computer, or a free standing kiosk.

A tablet computer may be suitable for providers who want a portable solution. For example, a portable handheld tablet computer may be useful in hospitals, where patients may not be in a state to use a desktop computer.

A desktop computer may be suitable in an office environment, where patients can meet with an approved representative for clinical trials.

A kiosk may be suitable in an office environment as well, where patients can complete initial check-in by themselves at the kiosk and then work with an approved representative on the informed consent.

In an example embodiment, computer 16 includes input device 32 and display 34, which may be combined as a touch or digitizer screen, and speakers 33. Computer 16 may include additional peripherals for recording patient identifying information in order to satisfy the local security policy, such as a card reader 36 for reading patient identification cards and/or a finger print capture device for capturing digitized finger prints or other biometric readers. Additional peripherals may include signature capture device 38 for recording patient signatures, printer 40 for printing consent forms, and scanner 42 for capturing signed printed consent forms. Peripherals 36, 38, and 40 may alternatively be network peripherals.

Computer 16 may be located in the lounge, or near an entrance to the healthcare provider office, at a receptionist desk, or other location in a healthcare facility.

Data store 18 stores consent files 46. Consent files 46 may include completed and uncompleted consent forms. Consent files 46 may also include audio/video files, help files, and other files that make an informed consent session more informative to the patient.

Store 18 may also store a consent database 54 for tracking informed consent events. Consent database 54 includes records of patients participating in clinical trials and other procedures, as well as reference identification information of authorized witnesses.

Those involved in experimental procedures may easily recall informed consents and informed consent process information in the event that legal challenges arise. Data store 18 may implement information security policies, to ensure privacy of participant data.

Store 18 may include a data server including permanent storage, such as hard disk drives. Store 18, CVM system 14, and healthcare information system 12 may be combined into one or more computer systems.

Healthcare system 10 may also include office computers 44 at the front desk and in examination rooms. Authorized office employees at the front desk may access patient records 21 in healthcare information system 12 and consent files 46 in store 18 through any of office computers 44.

Healthcare information system 12, customer value management CVM system 14, computer 16, store 18, and office computers 44 may all be connected via a network 48. Network 48 may be a wired or wireless network.

If healthcare information system 12 is located externally, CVM system 14 may alternatively access healthcare information system 12 using several methods, including messaging, files, etc. A common standard for exchanging healthcare data between health care information systems is HL7. CVM system 14 can receive patient information and schedule information via messages formatted in HL7 from health care information system 12. Alternatively, it can receive patient information via a flat file in a comma separated value (CSV) format or other formats.

In an example encounter, a patient arrives at a healthcare provider office for a blood sugar test. The patient checks in at computer 16. Patient check-in software 52 contacts CVM system 14 and CVM system 14 in turn contacts the provider's healthcare information system 12 to compare identification information provided by the patient to reference identification information in the patient's record 21.

A healthcare provider representative determines that the patient is a candidate for participating in a clinical trial of a new blood sugar testing protocol. The representative briefs the patient on the clinical trial and asks the patient if the patient is interested in participating.

From computer 16, the representative executes informed consent software 52, which contacts CVM system 14. CVM system 14 initiates and a workflow 28 for an informed consent session. CVM system 14 calls up one or more consent files 46 associated with the clinical trial, such as a consent form and clinical trial description information, and sends consent files 46 to informed consent software 52. Informed consent software 52 presents consent files 46 and records an informed consent from the patient.

Of course, after reviewing the consent form and the information about the clinical trial, the patient may decline to participate in the clinical trial.

Following recording of the patient's decision to participate or not to participate in the clinical trial, informed consent software 52 displays a prompt for one or more witnesses to sign in and provide signatures. CVM system 14 compares witness identification information to reference identification information in consent database 54. Witness staff are educated about the details of the clinical trial and are authorized to witness the patient's informed consent.

The role of the representative during the informed consent session may vary. For example, the representative may verbally present consent information to the patient, leaving informed consent software to record patient acceptance and witness verification, or the representative may just answer questions, leaving the patient interaction with computer 16 to be more self-service oriented.

The types of consent files 46 may vary along with representative involvement. An informed consent session that includes multimedia consent files 46 with voice and animation features may be more informative to a patient and require less representative involvement.

Informed consent may be captured in various ways. For example, informed consent may be recorded by having the patient respond to a "yes or no" prompt from informed consent software 52. As another example, informed consent software 52 may record informed consent by capturing a patient signature through signature capture device 38. As yet another example, informed consent software 52 may print a consent form for conventional signing. Witness signatures may be recorded in a similar manner.

Regardless of the method of recording informed consent, CVM system 14 stores information recorded during the informed consent session in store 18. For example, CVM system 14 may update patient records in consent database 54 to reflect that an informed consent has been processed. CVM system 14 may additionally store some of the information in the patient's record 21. Printed and signed consent forms are captured by scanning using scanner 42.

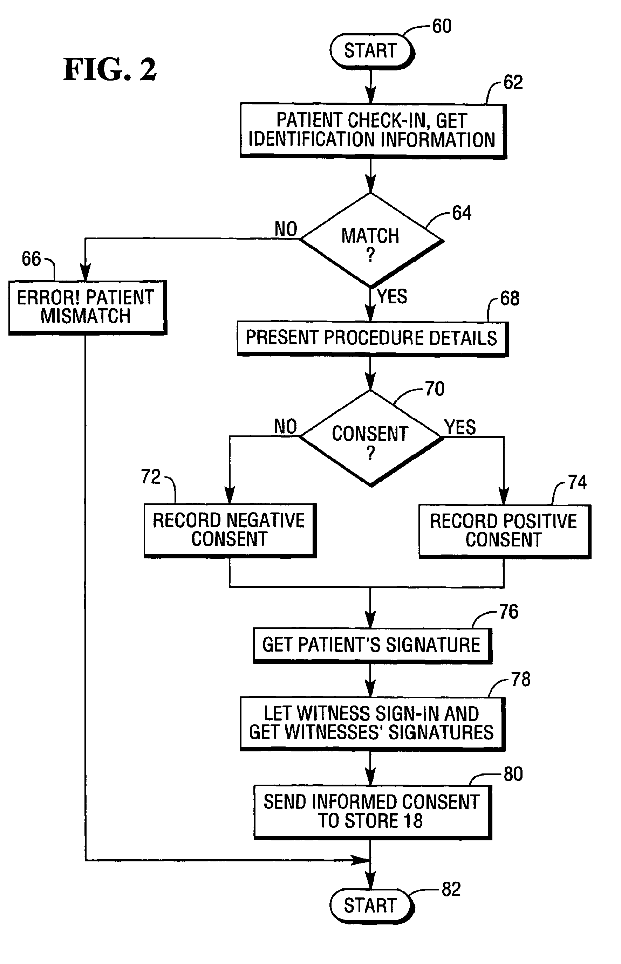

With reference to Fig. 2, the example informed consent session is further illustrated beginning with START 60.

In step 62, patient check-in software 52 records patient identification information during a check-in process. For example, the patient may be required to provide information such as a name, a phone number, and a date of birth. Further identification credentials, such as an identity number or identity card can be added to the process above for further security. They can be used in addition to, or in place of, other identification credentials, as dictated by the local security policy. CVM system 14 executes record identifying software 24 to attempt to match patient records 21 to patient identifying information presented by the patient.

In step 64, patient check-in software 52 receives an indication from CVM system 14 whether record identifying software 24 obtained a match. If so, operation proceeds to step 68. Otherwise, operation proceeds to step 66.

In step 66, patient check-in software 52 displays an indication that a match was not obtained. Patient check-in software 52 may direct the patient to a receptionist. Operation ends at step 82 until the patient can rectify the identification problem with the receptionist or other healthcare provider staff.

In step 68, the patient interacts with the informed consent software 54, which presents details of the clinical procedure to the patient. Informed consent software 54 may present the information in a variety of ways. Informed consent software 54 may present the consent details which can include several substeps or screens of information. With out loss of generality, the workflow and the screens presented in the substeps can be customized based on the procedure and the consent in context. Informed consent software 54 obtains consent files 46, including a consent form, information explaining the procedure, and possibly additional files, such as multi-media files, from data store 18.

In step 70, informed consent software 54 prompts the patient to provide either positive or negative consent to participate in the clinical procedure. If the patient provides a positive consent, operation proceeds to step 74. Otherwise, operation proceeds to step 72.

In step 72, informed consent software 54 records a negative consent.

In step 74, informed consent software 54 records a positive consent.

In step 76, informed consent software 54 prompts the patient to provide a signature. Informed consent software 54 may capture the signature using signature capture device 38. If computer 16 is a tablet computer, informed consent software 54 may capture the signature through a touch or digitizer screen of the tablet computer.

In step 78, informed consent software 54 prompts a witness or a plurality of witnesses to sign-in and provide signatures.

CVM system 14 compares witness identification information to reference identification information in consent database 54.

In step 80, informed consent software 52 sends all information collected during the informed consent session for storage in data store 18.

Advantageously, system 10 facilitates electronic recording and storage of informed consents at the point of care. This is a tremendous improvement over the current practice of manually obtaining the informed consent on paper, which is laborious, expensive, slow and difficult to track.

Although particular reference has been made to certain embodiments, variations and modifications are also envisioned within the scope of the following claims.

## Claims

1. A method of obtaining informed consent comprising:
(a) obtaining electronic informed consent information associated with a medical procedure by a computer;
(b) presenting the informed consent information to a prospective participant of the medical procedure by the computer; and
(c) electronically storing an informed consent from the prospective participant by the computer.

2. The method of claim 1, wherein step (b) comprises:
(b-1) displaying the electronic informed consent information on a display coupled to the computer.

3. The method of claim 2, wherein step (c) comprises:
(c-1) capturing
a first signature of the prospective participant by the computer; and
a second signature of a witness by the computer; and
(c-2) electronically storing the response with captured first and second signatures.

4. The method of any preceding claim, wherein step (c) comprises:
(c-1) scanning a printed and signed informed consent form by a scanner coupled to the computer; and
(c-2) electronically storing a scanned informed consent form.

5. The method of any preceding claim, wherein step (d) comprises:
(d-1) obtaining files containing the additional information by the computer; and
(d-1) displaying the additional information from the files.

6. The method of any preceding claim, wherein the files comprise multimedia files.

7. The method of any preceding claim, wherein step (b) occurs over more than one session with an authorized representative associated with the medical procedure.

8. The method of claim 7, further comprising:
logging progress during a first session in completing step (b); and
resuming step (b) at a subsequent session.

9. A system for obtaining informed consents comprising:
a computer for obtaining electronic informed consent information associated with a medical procedure, for presenting the informed consent information to a prospective participant of the medical procedure, and for electronically storing an informed consent from the prospective participant by the computer.

10. The system of claim 9, wherein the computer displays the informed consent information.

11. The system of claim 10, wherein the computer displays additional information to explain the medical procedure.

12. The system of any of claims 9 to 11, wherein the computer executes a multimedia file during presenting of the informed consent information.

13. The system of claim 12, wherein the computer stores the informed consent and the first and second signatures in an electronic store.

14. The system of any of claims 9 to 13, wherein the computer prints an informed consent form and captures a signed informed consent form signed by the prospective participant and a witness.

15. The system of claim 14, wherein the computer stores a captured signed informed consent form including the informed consent and the first and second signatures in an electronic store.
